# EUROPÄISCHE PATENTSCHRIFT

(11) **EP 2 892 412 B1**
(45) Veröffentlichungstag und Bekanntmachung des Hinweises auf die Patenterteilung: **06.07.2016**
(21) Anmeldenummer: 13740210.3
(22) Anmeldetag: 18.07.2013
(51) Int. Cl.: A61B 1/00, A61B 1/05, G02B 23/24, H04N 5/225

(54) **VIDEOENDOSKOP**
VIDEO ENDOSCOPE
ENDOSCOPE VIDÉO

(30) Priorität: 05.09.2012 DE 102012017499
(43) Veröffentlichungstag der Anmeldung: 15.07.2015
(73) Patentinhaber: Olympus Winter&Ibe GmbH, 22045 Hamburg (DE)
(72) Erfinder: STÜHLE, Sebastian, 22303 Hamburg (DE); JUNGBAUER, Sebastian, 22767 Hamburg (DE)
(74) Vertreter: Hausfeld, Norbert
(86) Internationale Anmeldenummer: PCT/EP2013/002141
(87) Internationale Veröffentlichungsnummer: WO 2014/037069

(56) Entgegenhaltungen:
- DE-A1- 19 806 984
- DE-A1-102010 044 786
- DE-B3-102004 023 866
- JP-A- H01 222 579
- US-A- 4 831 456

## Beschreibung

Die Erfindung betrifft ein Videoendoskop der im Oberbegriff des Anspruches 1 genannten Art.

Die DE 10 2004 023 866 B3 und die DE 10 2010 044 786 A1 zeigen gattungsgemäße Videoendoskope, bei denen der Bildsensor jeweils von einem starren Träger gehalten wird, der im Falle der erstgenannten Schrift als formsteife Platine und im Falle der zweitgenannten Schrift als stabförmiger Trägerkörper mit auf der Oberfläche angeordneten Leiterbahnen ausgebildet ist. An dem dem Bildsensor gegenüberliegenden Ende des Trägers ist dieser in beiden Fällen mit die Verschlusswand durchsetzenden Stiften verbunden, welche die Verschlusswand nach außen überragen und dort zum Anschluss der Leiterbahnen kontaktierbar sind.

Bei beiden bekannten Konstruktionen treten die in den Raum hineinragenden Enden der Stifte rechtwinklig in die dortige Stirnfläche des Trägers ein und können dort in herkömmlicher Löttechnik befestigt werden. Das erfordert aufwändige, kostenintensive Herstellungsschritte.

Die Aufgabe der vorliegenden Erfindung besteht darin, das eingangs genannte Videoendoskop konstruktiv und insbesondere hinsichtlich der Herstellungskosten zu verbessern.

Diese Aufgabe wird mit den Merkmalen des Kennzeichnungsteiles des Anspruches 1 gelöst.

Erfindungsgemäß trifft der Oberflächenbereich des Trägers, auf dem sich eine Leiterbahn befindet, die Verschlusswand an der Stelle, an der ein Stift nach innen hindurchragt. Dabei liegt der Stift der Oberfläche des Trägers parallel an. Daraus ergeben sich neue Möglichkeiten zur Gestaltung der Kontaktierung, so dass der Stift mit der Leiterbahn einfacher und insbesondere großflächiger kontaktiert werden kann. Damit lassen sich die Fertigungskosten senken und Kontaktierungsprobleme vermeiden.

Vorteilhaft gemäß Anspruch 2 ist der Winkel zwischen dem Oberflächenbereich und der Innenwand 90°. Dabei kann der Stift in der üblichen Weise senkrecht die Verschlusswand durchlaufen, um dann genau parallel zur Oberfläche des ebenfalls in üblicher Weise senkrecht zur Verschlusswand stehenden Trägers an diesem anliegen zu können. Damit ergibt sich die Möglichkeit großflächiger Kontaktierung zwischen Stift und Leiterbahn, wobei die Kontaktierungsfläche weitgehend beliebig über die Länge des kontaktierten Stiftbereiches wählbar ist.

Vorteilhaft sind dabei die Merkmale des Anspruches 3 vorgesehen. Es ergibt sich daraus die bewährte Konstruktion der DE 10 2010 044 786 A1.

Vorteilhaft gemäß Anspruch 4 ist die Kontaktierung zwischen Leiterbahn und Stift so ausgebildet, dass die Leiterbahn über den Stift hinwegläuft. Daraus ergibt sich insbesondere die Möglichkeit, die Leiterbahn in einer der üblichen Herstellungsarten auf der Oberfläche des Trägers und auf dem Stift anzubringen, z. B. als Metallschicht, die z. B. im Vakuum aufgedampft, bzw. elektrolytisch abgeschieden wird. Damit ergibt sich eine kostengünstige und besonders sichere Kontaktierung.

Der Träger kann, wie sich aus den eingangs genannten Schriften ergibt, z. B. über die Stifte mit der Verschlusswand verbunden sein. Vorteilhaft gemäß Anspruch 5 ist er jedoch direkt an der Verschlusswand befestigt. Das ermöglicht eine sehr sichere und feste Verbindung. Die Kontaktierungsstellen müssen nicht die mechanische Verbindung gewährleisten und werden somit entlastet.

Träger und Verschlusswand müssen aus isolierenden Materialien gefertigt sein, da sie die Leiterbahnen und die Stifte tragen und dabei Kurzschlüsse vermeiden müssen. Vorteilhaft gemäß Anspruch 6 sind Träger und Verschlusswand einstückig ausgebildet und somit aus demselben Material, beispielsweise einer geeigneten Keramik oder einem geeigneten Kunststoff. Durch die einstückige Ausbildung wird der nachträgliche Verbindungsschritt zwischen den beiden Teilen eingespart und es ergibt sich eine sehr feste Verbindung. Auch die Herstellungskosten können auf diese Weise reduziert werden. Die Herstellung kann z. B. in einem Kunststoffgussverfahren oder in einem Keramiksinterverfahren erfolgen, wobei die die Verschlusswand durchlaufenden Stifte bei der Herstellung eingeformt werden.

Das Systemrohr besteht üblicherweise aus Metall, also einem vom Material des Trägers und der Verschlusswand abweichenden Material. Vorzugsweise gemäß Anspruch 7 sind jedoch alle drei Teile einstückig und somit auch aus demselben isolierenden Material gebildet. Damit ergibt sich die Möglichkeit, die gesamte Anordnung einstückig herzustellen, was fertigungstechnische Vorteile ergibt. Die Verschlusswand kann einstückig ausgebildet sein, kann jedoch auch vorteilhaft gemäß Anspruch 8 in ein distales und ein proximales Stück geteilt sein, von denen das distale Teilstück am Träger befestigt ist. An der Trennebene können diese Stücke aneinander montiert werden. Es ergeben sich hieraus Fertigungs- und Kostenvorteile.

Nach Anspruch 9 wird bei der Herstellung des Videoendoskopes vorteilhaft so vorgegangen, dass an einer Kontaktierungsstelle zwischen Stift und Leiterbahn zunächst der Stift auf der Oberfläche des Trägers anliegend angeordnet wird und sodann die Leiterbahn auf der Oberfläche des Trägers und auf dem Stift aufgebracht wird. Damit kann in einem zur Herstellung der Leiterbahn geeigneten Schichtauftragungsverfahren gleichzeitig mit deren Anbringung auf dem Träger die Kontaktierung mit dem Stift hergestellt werden, ohne dass dazu ein gesonderter Fertigungsschritt, wie z. B. eine Verlötung erforderlich wäre.

In der Zeichnung ist die Erfindung beispielsweise und schematisch dargestellt. Es zeigen:
- Fig. 1: einen Längsschnitt durch ein erfindungsgemäßes Videoendoskop mit einer Verschlusswand an einem Ende,
- Fig. 2: einen vergrößerten Ausschnitt aus Fig. 1 im Bereich der Verschlusswand,
- Fig. 3: eine Darstellung entsprechend Fig. 2 in anderer Ausführungsform,
- Fig. 4: die Konstruktion der Fig. 2, jedoch ohne das Systemrohr,
- Fig. 5: die Konstruktion der Fig. 4 mit in zwei Stücke geteilter Verschlusswand,
- Fig. 6: einen Schnitt nach Linie 6 - 6 in Fig. 5,
- Fig. 7: die Konstruktion der Fig. 5 in einer Ausführungsvariante und
- Fig. 8: einen Schnitt nach Linie 8 - 8 in Fig. 7.

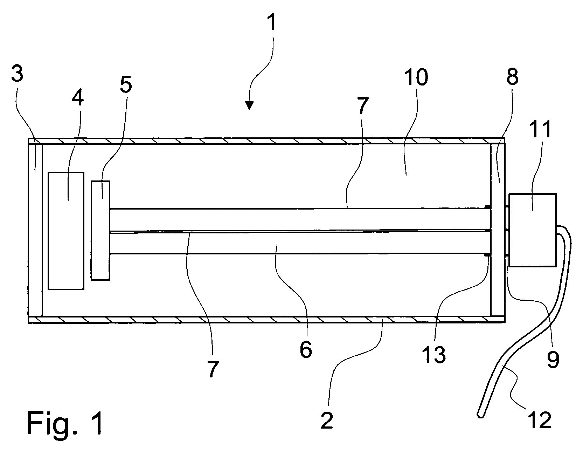

Fig. 1 zeigt ein Videoendoskop 1, das in der dargestellten Form selbstständig nutzbar ist oder auch in ein endoskopisches Gerät eingebaut werden kann.

Das dargestellte Videoendoskop 1 weist ein langgestrecktes Systemrohr 2 von im Ausführungsbeispiel rundem Querschnitt auf, das in üblicher Ausbildung aus einem geeigneten rostfreien Stahl hergestellt ist. Am distalen Ende ist das Systemrohr 2 mit einem Fenster 3 verschlossen, an dessen Innenseite ein Objektiv 4 angeordnet ist. Ein Bildsensor 5 betrachtet das distal vom Videoendoskop 1 liegende Arbeitsgebiet durch das Objektiv 4 und das Fenster 3 hindurch.

Der Bildsensor 5 ist am distalen Ende eines stabförmigen Trägers 6 angeordnet, der im Ausführungsbeispiel von rundem Querschnitt ist. Er trägt auf seiner Oberfläche mehrere in Längsrichtung des Trägers 6 erstreckte Leiterbahnen 7. In der Zeichnung ist eine Ausführungsform dargestellt, bei der der Träger 6 vier um 90° umfangsbeabstandete Leiterbahnen trägt.

Am proximalen Ende des Systemrohres 2 ist dieses mit einer Verschlusswand 8 verschlossen, an der, wie die Fig. 1 zeigt, der Träger 6 anstoßend befestigt ist. Die Verschlusswand 8 wird von Stiften 9 durchsetzt, die an ihren, in den Innenraum 10 hineinragenden Enden mit den Leiterbahnen 7 kontaktiert sind.

Die nach außen aus der Verschlusswand 8 herausragenden Enden der Stifte 9 lassen sich z. B. mit dem in Fig. 1 dargestellten Stecker 11 kontaktieren, der über ein Kabel 12 an einer nicht dargestellten Bildverarbeitungseinrichtung angeschlossen ist, die somit über mehrere elektrische Leitungen mit dem Bildsensor 5 verbunden ist. Über diese Leitungen kann der Bildsensor z. B. mit Strom versorgt werden und kann sein Videosignal nach außen abgeben.

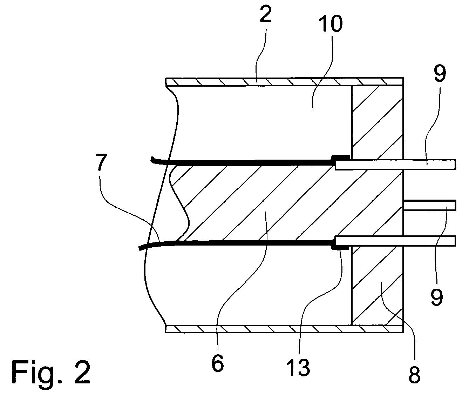

Wie bereits erwähnt, ist der Träger 6 am proximalen Ende mit der Verschlusswand 8 verbunden. Wie Fig. 2 im Einzelnen zeigt, können dabei diese beiden Teile einstückig ausgebildet sein und somit aus demselben Material, z. B. einem geeigneten Kunststoff oder einer geeigneten Keramik. Wichtige Anforderungen sind elektrisches Isolationsvermögen, damit die verschiedenen Stifte 9, bzw. Leiterbahnen 7 gut gegeneinander isoliert sind.

Fig. 2 zeigt, dass der Träger 6 mit seiner Achse senkrecht zur Ebene der Verschlusswand 8 steht. Damit ergibt sich, dass die Oberfläche des Trägers 6 überall im rechten Winkel auf die Innenseite der Verschlusswand 8 trifft.

Wie Fig. 2 zeigt, stehen die Stifte 9 zueinander parallel und senkrecht zur Verschlusswand 8. Sie stehen daher parallel zur Achse des Trägers 6 und können bei geeigneter Anordnung, wie in Fig. 2 dargestellt, mit ihren innenliegenden, die Verschlusswand 8 in den Innenraum 10 hinein überragenden Enden parallel und flach auf der Außenseite des Trägers 6 liegend angeordnet sein. Wie Fig. 2 zeigt, können sie auch etwas in die Oberfläche des Trägers 6 eingebettet sein.

Wie Fig. 2 ferner zeigt, verlaufen die Leiterbahnen 7 auf der Oberfläche des Trägers 6 und im Endbereich an der Kontaktierungsstelle 13 über die Stifte 9 hinweg.

Die Herstellung der Konstruktion der Fig. 2 erfolgt vorzugsweise so, dass zunächst die Einheit aus Träger 6 und Verschlusswand 8 einstückig als Kunststoffgusskörper, bzw. als Keramiksinterkörper hergestellt wird. Dabei werden die Stifte 9 eingearbeitet, die z. B. in der Kunststoffspritzform zum Umspritzen gehalten werden. Im Falle der Keramiksinterung können die Stifte im Grünkörper, also in dem noch nicht gebrannten Material angebracht werden. Dabei ist für eine gasdichte Einbettung zu sorgen.

Anschließend werden die Leiterbahnen aufgebracht, die in bekannter Weise elektrochemisch oder durch Aufdampfen, bzw. Sputtern aufgebracht werden können. Dabei werden die Leiterbahnen gleichzeitig auf der Oberfläche des Trägers 6 und auf den Stiften 9 abgeschieden, wodurch sich eine besonders sichere Kontaktierung ergibt.

Die in Fig. 2 nicht dargestellten Teile des Videoendoskopes müssen montiert werden und es wird abschließend das Systemrohr 2 übergeschoben und am Rand der Verschlusswand 8 abgedichtet befestigt, z. B. durch Verklebung, Verlötung, Verpressung oder dergleichen.

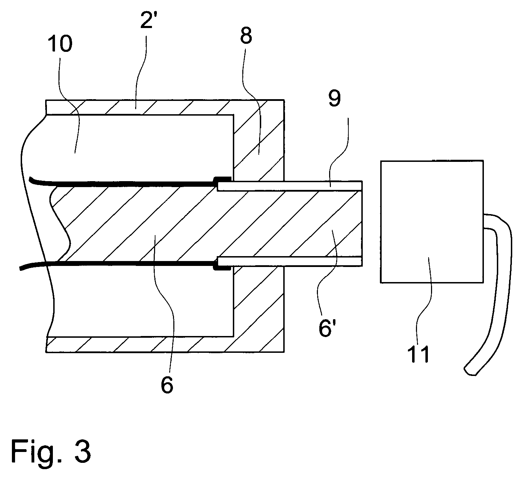

Fig. 3 zeigt eine Ausführungsvariante zu Fig. 2. Dabei ist das Systemrohr 2 durch ein Rohr 2' ersetzt, das einstückig mit dem Träger 6 und der Verschlusswand 8 ausgebildet ist. Es kann hier also die gesamte Grundkonstruktion einstückig hergestellt werden. Dadurch werden Fertigungsprobleme, z. B. mit der Dichtigkeit, verringert und Kosten gesenkt.

Vergleicht man mit Fig. 2, so sieht man, dass bei der Ausführungsform der Fig. 3 ein entsprechend dem Träger 6 gestalteter Stutzen 6' ausserhalb der Verschlusswand 8 in der Flucht des Trägers 6 angeordnet ist. Auf diesem sitzen die Stifte 9 ebenso auf der Außenseite angeordnet, wie dies im Innenraum 10 beim Träger 6 der Fall ist.

Die die Verschlusswand 8 nach außen überragende Anordnung mit dem Stutzen 6' und den anliegenden Stiften 9 ergibt eine Konstruktion, die gut als männlicher

Stecker geeignet ist, der mit dem weiblichen Stecker 11 gekuppelt werden kann. Der äußere männliche Stecker 6', 9, ist sehr stabil und sichert die Konstruktion z.B. gegen Verbiegen der Stifte.

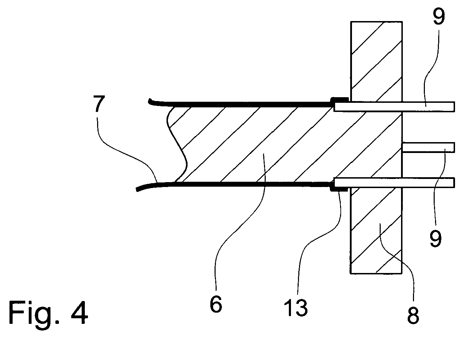

In den Ausführungsformen der Figuren 2 und 3 ist die Verschlusswand 8 einstückig ausgebildet. Um dies zu verdeutlichen ist in Fig. 4 noch einmal der innere Teil der Konstruktion der Fig. 2 dargestellt, also bei weggelassenem Systemrohr 2. Bei der Montage wird die in Fig. 4 dargestellte Baueinheit zunächst fertiggestellt und dann gemäß Fig. 2 mit dem Systemrohr 2 verbunden.

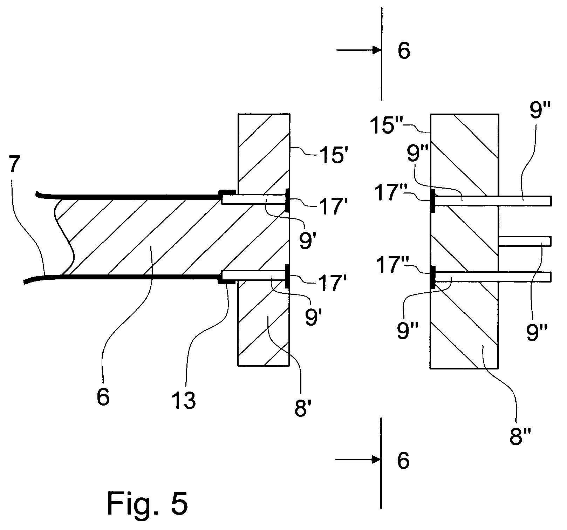

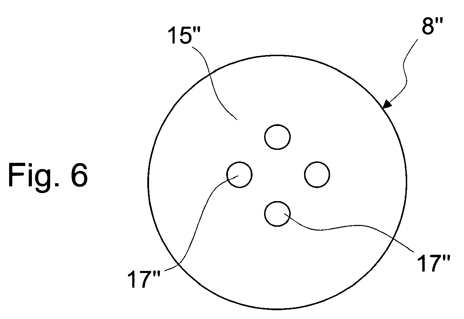

Die Figuren 5 und 6 zeigen eine Ausführungsvariante, bei der gegenüber der in Fig. 4 dargestellten Konstruktion ersichtlich ist, dass die Verschlusswand 8 der Fig. 4 nun in zwei Stücke 8' und 8" geteilt ist. Die Teilstücke 8' und 8" weisen gegeneinander gerichtete Planflächen 15' und 15" auf, mit denen die Teilstücke 8' und 8" aneinandersetzbar sind. Sie können dann z. B. durch Verklebung verbunden werden und dann anschließend in der zuvor beschriebenen Weise in das Systemrohr 2 eingesetzt und dort randverlötet werden.

Wie Fig. 5 zeigt, sind auch die die Verschlusswand durchsetzenden Stifte 9 jeweils in Stücke 9' und 9" geteilt. Die Enden der Stiftteile 9' und 9", die in den Planflächen 15' und 15" liege, tragen dort Kontaktplatten 17' und 17", die bei der Befestigung der Teilstücke 8' und 8" in Berührung kommen und miteinander verlötet oder leitfähig verklebt werden können.

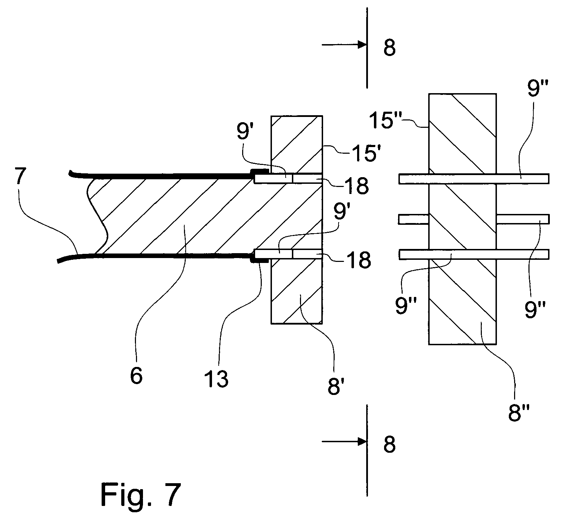

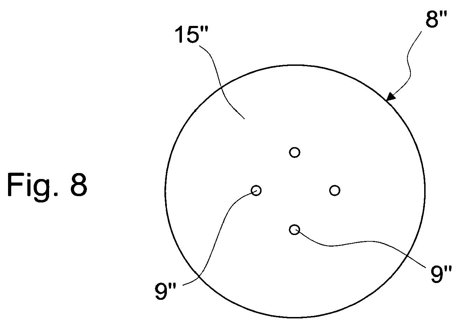

Die Figuren 7 und 8 zeigen eine Ausführungsvariante zur Ausführungsform der Figuren 5 und 6. Es werden dieselben Bezugszeichen verwendet. Die konstruktiven Unterschiede sind wie folgt:
Das distale Teilstück 8' der Verschlusswand ist im Durchmesser kleiner. Das ist für die vorliegenden Zwecke ausreichend. Die Befestigung im Systemrohr 2 wird am Teilstück 8" vorgenommen.

Die im distalen Teilstück 8' liegenden Stücke 9' der Stifte sind unter die Planfläche 15' versenkt. Die Stiftteile 9" im proximalen Teilstück 8" sind über die Planfläche 15" in distaler Richtung vorspringend ausgebildet und können beim Befestigen der Teilstücke 8' und 8" aneinander in Bohrungen 18 eintauchen, die oberhalb der Stiftteile 9' ausgebildet sind.

Es ergibt sich dabei ein bei der Montage hilfreicher Formschlusseingriff der Stiftteile 9' in die Bohrungen 18. Am Grunde der Bohrungen 18 treffen sich die Stiftteile 9' und 9" und können dort leitend verklebt oder verlötet werden.

## Patentansprüche

1. Videoendoskop (1) mit einem von einem Systemrohr (2) gasdicht umschlossenen Innenraum (10), in dem ein durch ein Fenster (3) aus dem Innenraum (10) herausblickender Bildsensor (5) an einem Träger (6) angeordnet ist, auf dem mindestens eine den Bildsensor (5) elektrisch anschließende Leiterbahn (7) angeordnet ist, wobei der Innenraum (10) bereichsweise von einer Verschlusswand (8) begrenzt ist, welche von einem im Innenraum (10) auf dem Träger (6) mit der Leiterbahn (7) kontaktierten Stift (9) abgedichtet durchlaufen ist, **dadurch gekennzeichnet, dass** der Träger (6) derart gegen die Innenseite der Verschlusswand (8) stößt, dass sein die Leiterbahn tragender Oberflächenbereich die Verschlusswand (8) an der Stelle des Stiftes (9) unter einem solchen Winkel trifft, dass der in den Innenraum (10) ragende Endbereich des Stiftes (9) dem Oberflächenbereich des Trägers (6) parallel anliegt und die Leiterbahn (7) kontaktiert.

2. Videoendoskop nach Anspruch 1, **dadurch gekennzeichnet, dass** der Oberflächenbereich des Trägers (6) die Verschlusswand (8) unter einem rechten Winkel trifft.

3. Videoendoskop nach Anspruch 2, **dadurch gekennzeichnet, dass** der Träger (6) als senkrecht zur Verschlusswand (8) stehender Stab ausgebildet ist, wobei der Stift (9) die Verschlusswand (8) parallel zur Achse des Stabes (6) durchsetzt.

4. Videoendoskop nach einem der vorhergehenden Ansprüche, **dadurch gekennzeichnet, dass** die Leiterbahn (7) den Stift (9) überlaufend ausgebildet ist.

5. Videoendoskop nach einem der vorhergehenden Ansprüche, **dadurch gekennzeichnet, dass** der Träger (6) an der Verschlusswand (8) befestigt ist.

6. Videoendoskop nach Anspruch 5, **dadurch gekennzeichnet, dass** der Träger (6) mit der Verschlusswand (8) einstückig ausgebildet ist.

7. Videoendoskop nach Anspruch 6, **dadurch gekennzeichnet, dass** die Verschlusswand (8) mit dem Systemrohr (2) einstückig ausgebildet ist.

8. Videoendoskop nach einem der vorhergehenden Ansprüche, **dadurch gekennzeichnet, dass** die Verschlusswand (8) an einer quer zum Stift (9) liegenden Ebene (15', 15") in zwei Teilstücke (8', 8") geteilt ist, von denen das distale Teilstück (8') am Träger (6) befestigt ist.

9. Verfahren zur Herstellung eines Videoendoskopes (1) nach Anspruch 4,
**dadurch gekennzeichnet, dass** der Stift (9) mit seinem in den Innenraum ragenden Endbereich der Oberfläche des Trägers (6) anliegend angeordnet wird und dass dann die Leiterbahn (7) auf der Oberfläche des Trägers (6) und auf dem Stift (9) aufgebracht wird.

## Claims

1. Video endoscope (1) comprising an interior (10), which is enclosed by a system tube (2) in a gas-tight manner and in which an image sensor (5), which is able to view the outside from the interior (10) through a window (3), is arranged on a support (6), on which at least one strip conductor (7), which electrically connects the image sensor (5), is arranged, wherein, in some areas, the interior (10) is delimited by a closing wall (8), through which a pin (9), which is in contact with the strip conductor (7) on the support (6) in the interior (10), extends in a sealed manner,
**characterised in that**
- the support (6) abuts the inner surface of the closing wall (8) in such a manner that the surface region of said support that supports the strip conductor touches the closing wall (8) at such an angle at the point where the pin (9) is located such that the end region of the pin (9), which protrudes into the interior (10), abuts the surface region of the support (6) in a parallel manner and contacts the strip conductor (7).

2. Video endoscope according to claim 1, **characterised in that** the surface region of the support (6) touches the closing wall (8) at a right angle.

3. Video endoscope according to claim 2, **characterised in that** the support (6) is embodied as bar, which is vertical to the closing wall (8), wherein the pin (9) permeates the closing wall (8) parallel to the axis of the bar (6).

4. Video endoscope according to any one of the preceding claims, **characterised in that** the strip conductor (7) is embodied so as to overtravel the pin (9).

5. Video endoscope according to any one of the preceding claims, **characterised in that** the support (6) is fastened to the closing wall (8).

6. Video endoscope according to claim 5, **characterised in that** the support (6) is formed integrally with the closing wall (8).

7. Video endoscope according to claim 6, **characterised in that** the closing wall (8) is formed integrally with the system tube (2).

8. Video endoscope according to any one of the preceding claims, **characterised in that**, at a plane (15', 15"), which is located at right angles to the pin (9), the closing wall (8) is divided into two partial pieces (8', 8"), of which the distal partial piece (8') is fastened to the support (6).

9. Method for producing a video endoscope (1) according to claim 4, **characterised in that** the pin (9) is arranged so that its end region, which protrudes into the interior, abuts the surface of the support (6) and that the strip conductor (7) is then applied to the surface of the support (6) and to the pin (9).

## Revendications

1. Endoscope vidéo (1) doté d'un espace intérieur (10) entouré de manière étanche au gaz par un tube formant système (2) dans lequel un capteur d'image (5) opérant à partir de l'espace intérieur (10) à travers une fenêtre (3) est disposé sur un support (6) sur lequel est agencée au moins une piste conductrice (7) assurant la connexion électrique du capteur d'image (5), l'espace intérieur (10) étant en partie délimité par une paroi d'obturation (8), laquelle est traversée, par un passage étanchéifié, par une broche (9) qui, dans l'espace intérieur (10), est en contact avec la piste conductrice (7) sur le support (6), **caractérisé en ce que** le support (6) butte contre la face intérieure de la paroi d'obturation (8) de façon telle que la zone de sa surface qui porte la piste conductrice touche la paroi d'obturation (8) à l'endroit de la broche (9) sous un angle tel que la section d'extrémité de la broche (9) qui pénètre dans l'espace intérieur (10) est parallèlement adjacente à la zone de surface du support (6) et contacte la piste conductrice (7).

2. Endoscope vidéo selon la revendication 1, **caractérisée en ce que** la zone de surface du support (6) butte contre la paroi d'obturation (8) selon un angle droit.

3. Endoscope vidéo selon la revendication 2, **caractérisée en ce que** le support (6) est réalisé sous forme de tige perpendiculaire à la paroi d'obturation (8), la broche (9) traversant la paroi d'obturation (8) parallèlement à l'axe de la tige (6).

4. Endoscope vidéo selon l'une des revendications précédentes, **caractérisé en ce que** la piste conductrice (7) est réalisée de façon à chevaucher la broche (9).

5. Endoscope vidéo selon l'une des revendications précédentes, **caractérisé en ce que** le support (6) est fixé à la paroi d'obturation (8).

6. Endoscope vidéo selon la revendication 5, **caractérisée en ce que** le support (6) est façonné d'un seul tenant avec la paroi d'obturation (8).

7. Endoscope vidéo selon la revendication 6, **caractérisée en ce que** la paroi d'obturation (8) est façonnée d'un seul tenant avec le tube formant système (2).

8. Endoscope vidéo selon l'une des revendications précédentes, **caractérisé en ce que** la paroi d'obturation (8) est subdivisée selon un plan (15', 15") transversal à la broche en deux éléments (8', 8"), l'élément distal (8') étant fixé au support (6).

9. Procédé de fabrication d'un endoscope vidéo (1) selon la revendication 4, **caractérisé en ce que** la broche (9) est agencée de façon à ce que sa section d'extrémité saillante dans l'espace intérieur soit adjacente à la surface du support (6) et que la piste conductrice (7) est alors appliquée sur la surface du support (6) et sur la broche (9).
